(19) Europäisches Patentamt
European Patent Office
Office européen des brevets

(11) Publication number: **0 154 587 B1**

# (12) EUROPEAN PATENT SPECIFICATION

(45) Date of publication of patent specification: **15.05.91**

(51) Int. Cl.[5]: **C12N 15/41**, C12N 15/70, C07H 21/04

(21) Application number: **85400369.6**

(22) Date of filing: **27.02.85**

## (54) Fragments of DNA which encode peptides capable of inducing in vivo the synthesis of anti-hepatitis A virus antibodies.

(30) Priority: **02.03.84 US 585818**

(43) Date of publication of application:
**11.09.85 Bulletin 85/37**

(45) Publication of the grant of the patent:
**15.05.91 Bulletin 91/20**

(84) Designated Contracting States:
**CH DE FR GB IT LI NL**

(56) References cited:
**EP-A- 0 061 740**
**EP-A- 0 138 704**
**WO-A-85/01517**

**CHEMICAL ABSTRACTS, vol. 102, no. 25, 25th June 1985, page 184, abstract no. 216279v, Columbus, Ohio, US; D.L. LINEMEYER et al.: "Molecular cloning and partial sequencing of hepatitis A viral cDNA**

**PROC. NAT. ACAD. SCI. USA, vol. 80, October 1983, pages 5885-5889, Biochemistry, Baltimore, US; J.R. TICEHURST et al.: "Molecular cloning and characterization of hepatitis A virus cDNA"**

(73) Proprietor: **MERCK & CO. INC.**
**126, East Lincoln Avenue P.O. Box 2000**
**Rahway New Jersey 07065-0900(US)**

(72) Inventor: **Linemeyer, David L.**
**526 Clark Street**
**Westfield New Jersey 07090(US)**
Inventor: **Menke, John G.**
**21 Yorktown Drive**
**Manapalan New Jersey 07726(US)**
Inventor: **Reuben, Roberts C.**
**R. D. No. 2, Box 74**
**Boonton New Jersey 07005(US)**
Inventor: **Mitra, Sudha W.**
**511th Village Drive**
**Avenel New Jersey 07001(US)**

(74) Representative: **Ahner, Francis et al**
**CABINET REGIMBEAU, 26, avenue Kléber**
**F-75116 Paris(FR)**

EP 0 154 587 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

Hepatitis A is a liver disease which, although not commonly fatal, can induce long periods of debilitating illness. The disease is commonly spread by direct contact with an infected individual or by hepatitis A virus (HAV) contaminated drinking water and/or food.

The prior art does not identify the protein or proteins of HAV (hepatitis A virus) which induce neutralizing antibodies to this virus. One of the major drawbacks to examining the protective antigenicity of HAV proteins has been the lack of sufficient quantities of HAV and its polypeptide components. The virus is made in very small quantities in cell culture, has a limited animal host range, and is difficult to purify from infected cell cultures and animal tissues. Recently a patent application claiming the VP-1 structural protein of HAV prepared by isolation from the virus was filed by the assignee of this application and is presently copending as U.S.S.N. 541,836, filed October 14, 1983 (See EP-A-0138 704). VP-1 is recognized in our laboratories as being the main structural protein of HAV, and accordingly the most important for vaccine use. The appropriate dosage forms and regions are in this application, which is incorporated by reference.

Cloning of the genomic material of HAV and potential analysis of its sequence has recently been reported: Von Der Helm et al., J. Virological Methods 3 1981, 37-43; see also EPO 0061740, priority March 28, 1981 published October 6, 1982 disclosing the same work; and Ticehurst et al., Proc. National Academy Science USAVol. 80, pgs 5885-5889. October 1983.

The Von de Helm work describes preparation of cDNA and cloning of that cDNA into plasmids, and subsequent expression of HAV antigen, but no nucleotide sequence of the DNA or amino acid sequence of the corresponding proteins is described, nor is the identity of any of the viral proteins expressed known. In fact, the weak antigenic responses of the viral proteins obtained by Von de Helm et al. tend to indicate that most portions of the HAV genomic encoding the important antigenic proteins of HAV has not been successfully identified or used in the cloning experiments.

Ticehurst does report a partial nucleotide sequence from the 3' terminus of HAV, but does not appear to have identified that part of the sequence which encodes for the antigenic proteins. From our experiments, we believe that Ticehurst has not sequenced the antigenic portion; that the Ticehurst sequence work is far outside the antigenic region.

### OBJECTS OF THE INVENTION

It is an object of the present invention to provide a method for cloning the nucleotide sequences of HAV encoding the major antigenic proteins of HAV, including VP-1, VP-2, VP-3 and VP-4, especially VP-1. Another object is to determine the nucleotide sequences which encode the antigenic proteins, including VP-1. Yet another object is to provide a method for producing VP-1, VP-2, VP-3, VP-4, or parts thereof, by expression of cloned DNA in an appropriate host. Still another object is to provide a vector containing the VP-1, VP-2, VP-3 or VP-4 genes, or parts thereof, but especially a vector containing the VP-1, or part of VP-1, gene. Another object is to provide transformed hosts containing a vector containing the VP-1, VP-2, VP-3 or VP-4 genes, or parts thereof and being capable of expressing the peptide encoded by said genes or parts thereof. These and other objects of the present invention will be apparent from the following description.

### SUMMARY OF THE INVENTION

Fragments of DNA which code for immunogenic peptides VP-1, VP-2, VP-3 and VP-4 capable of inducing in vivo the formation of anti-Hepatitis A virus antibodies are provided and their nucleotide sequence determined. In other terms, the invention concerns fragments of DNA which code for the antigenic determinants on peptides normally encoded by the RNA of HAV or by corresponding double-stranded cDNA; these antigenic determinants being essential for the antigenic properties of the products of natural viral RNA expression.

### DETAILED DESCRIPTION

RNA was extracted from purified hepatitis A virus particles, annealed to a dT-tailed plasmid, and the complementary cDNA synthesized. This single stranded DNA was used as a template for synthesis of its complementary strand using DNA polymerase. The double stranded cDNA thus formed corresponded to at

least a portion of the HAV antigenic protein genes. It was modified to provide "sticky ends" and placed into an appropriate vector; suitable hosts include prokaryotic and eukaryotic organisms. These hosts were exposed to the resulting vector and those which stably incorporated the vector were identified and isolated.

Vector DNA was extracted from these hosts and this material was characterized. At least 5 fragments of cloned HAV DNA were sequenced to determine the nucleotide sequence of the region encoding the antigenic peptides, VP-1, VP-2, VP-3 and VP-4. The corresponding amino acid sequence encoded thereby was inferred.

Knowledge of the nucleotide sequences encoding the amino acid sequences of these peptides makes possible their synthesis as well as subunits and homologues and analogues thereof thus allowing determination of the relationship between structure and function. The peptides, which as noted above, do not form part of this invention, can be used in pharmaceutical compositions to make vaccines.

The HAV peptides of the present invention may be prepared from their constituent amino acids by standard methods of protein synthesis, e.g., Schroeder et al., "The Peptides", Vol. I, Academic Press, 1965, or Bodanszky et al., "Peptide Synthesis", Interscience Publishers 1966, or McOmie (ed.), "Protective Groups in Organic Chemistry", Plenum Press 1973, the disclosures of which are hereby incorporated by reference.

The peptides of HAV also may be prepared by recombinant DNA techniques by, for example, the isolation or preparation of appropriate DNA sequences and incorporation of these sequences into vectors in a suitable host and expression of the desired peptide therefrom. The use of recombinant DNA techniques is described in many published articles, for example, Maniatis et al., Molecular Cloning, A Laboratory Manual, Cold Spring Harbor, New York 1982, the disclosure of which is hereby incorporated by reference. Modification of the nucleotides coding for the peptides of the present invention according to known techniques permits the preparation via recombinant DNA techniques of peptides having altered amino acid sequences of the peptides of the present invention. Certain of these amino acids may be coded for by more than one triplet nucleotide sequence. It is to be understood that the disclosed nucleotide sequence is to include other codons coding for the same amino acid, e.g., the codons CGA and AGA each code for arginine.

Suitable hosts for expression of the HAV peptides include prokaryotic organisms such as E. coli and B. subtilis, and eukaryotic organisms such as Saccharomyces cerevisiae and Chinese hamster ovary cells. It is also to be understood that these proteins can be expressed directly in a mammalian species by means of appropriate expression vectors such as vaccinia, varicella zoster, adeno or herpes simplex viruses.

The following examples illustrate the present invention.

EXAMPLE 1

VIRUS RNA EXTRACTION

1.1 Hepatitis A virus particles (strain CR326), purified from virus-infected LLC-MK2 cells (a monkey kidney cell line) by CsCl gradient centrifugation, were disrupted at 65° C with 1% SDS, 20 mM EDTA and phenol extraction using 0.1M tris pH 7.4 - saturated phenol.

1.2 The aqueous layer of the extraction was further extracted twice with equal volumes of $CHCl_3$; isoamyl alcohol (24:1) and precipitated with 0.2 M sodium acetate pH 5.5 and 2 volumes of EtOH at -20° C.

1.3 The EtOH precipitate was collected by centrifugation and dissolved in $H_2O$.

EXAMPLE 2

PREPARATION OF cDNA CLONES FROM THE 3' END OF THE VIRAL GENOME

The initial 3' cDNA clones were prepared using the procedure of H. Okayama and P. Berg (Molecular and Cellular Biology 2: 161-170 (1982)). In brief, the viral RNA was annealed to dT-tailed plasmid (derived from pSV 0.71-0.86) and cDNA synthesized by reverse transcriptase using the dT-tailed linker (derived from pSV 0.19-0.32). Using the linker as a primer, the second cDNA strand was synthesized by DNA polymerase after removal of the RNA by RNase H. The cDNA was ligated and transformed into E. coli. The resulting clones were selected by resistance to ampicillin and screened by hybridization to a $^{32}P$-labeled HAV cDNA prepared from the viral RNA.

The largest viral insert obtained was approximately 2.3Kb in size and was called clone α 18. Restriction enzyme analysis of this clone demonstrated the presence of two Pvu II sites near the 5' end of the cloned insert. The cloned DNA was restricted with Pvu II and the 280 bp 5' proximal fragment was purified by agarose gel electrophoresis, visualized by ethidium bromide staining and electroeluted in dialysis tubing for use as a primer for further cDNA cloning.

EXAMPLE 3
PREPARATION OF cDNA CLONES BY PRIMER EXTENSION

3.1 The 280 bp Pvu II restriction fragment of cDNA clone $\alpha$ 18 was denatured by boiling for 10 min. and quick-cooling in ice-water, and then used as a primer for HAV cDNA synthesis. The denatured primer was either annealed or hybridized to the viral RNA. For annealing, 160 ng of denatured primer was added to approximately 1 $\mu$g of purified HAV RNA in 20 $\mu$l of $H_2O$ and heated to 70° C for 10 min. then 37° C for 15 min. For the hybridization, 140 ng of denatured primer was added to 1 $\mu$g of purified HAV RNA in 80% formamide, 0.4 M NaCl, 0.01 M PIPES pH 6.4, and 2 mM EDTA and incubated at 47° C for 3.5 hours. The formamide was removed by performing 4 sequential ethanol precipitations.
3.2 After annealing or hybridization of the primer to the RNA, the first strand of cDNA was synthesized using 50 mM Tris pH 8, 0.34 mM dCTP, 1 mM dGTP, dATP, dTTP, 10 mM 2-mercaptoethanol, 10 mM magnesium acetate, [$^{32}$P-$\alpha$]dCTP (8 $\mu$ Ci/10 $\mu$l), RNasin (7 units/10 $\mu$l), and AMV reverse transcriptase (16 units/l0 $\mu$l) by incubating at 42° C for 30 min. The reaction mix was extracted twice with phenol, chloroform extracted, and precipitated with ethanol.
3.3 The second strand of cDNA was then synthesized using 20 mM Tris pH 7.4, 5 mM $MgCl_2$, 10 mM $(NH_4)_2SO_4$, 100 mM KCl, 100 $\mu$g/ml BSA, 0.04 mM dATP, dGTP, dCTP and dTTP, 9 units/ml RNase H, and 1750 units/ml DNA polymerase I by incubating at 12° C for 60 min. and then 22° C for 60 min. The reaction mix was extracted twice with phenol and chloroform and ethanol precipitated.
3.4 The double-stranded cDNA was dC-tailed using terminal deoxynucleotidyl transferase, annealed to pBR 322 DNA which was dG-tailed at the PstI site, and transformed into competent E. coli strain RR-1. The resulting clones were selected for tetracycline resistant, ampicillin-sensitive growth and screened for positive hybridization to a $^{32}$P-labeled cDNA prepared from HAV RNA.

EXAMPLE 4
VERIFICATION OF HAV-SPECIFICITY OF cDNA CLONES

To insure the cDNA clones were generated from Hepatitis A genetic information, the clones were labeled with [$^{32}$P-$\alpha$] dCTP by nick translation and hybridized to uninfected and HAV-infected LLC-MK2 cellular RNA bound to nitrocellulose membrane filters. The cDNA clones hybridized only to the HAY-infected cell RNA.

EXAMPLE 5
DETERMINATION OF THE RELATIVE POSITION OF THE CLONED cDNAs AND ANALYSIS OF THE BASE SEQUENCE

The cloned cDNAs were analyzed by restriction enzyme digestion and by cross-hybridization of the clones to one another in order to locate the relative positions of five clones, T28-18, T28-123, T28-94, T28-71 and T28-77, as shown in Fig. 1. From this analysis one can deduce that over 90% of the viral genome has been cloned in overlapping cDNA clones starting at the 3' end of the genome. The restriction enzyme map of the HAV genome is that line marked "HAV" in Fig. 1. The location of the structural proteins on the polio genome is also shown in Fig. 1 as an indication of the probable predicted position on the HAY genome, since polio and HAV are related viruses.

EXAMPLE 6
ANALYSIS OF THE BASE SEQUENCE OF THE cDNA CLONES

DNA sequencing has been performed by two methods.

Chemical DNA sequencing has been performed by the Maxam and Gilbert technique on clones T28-18, T28-123, T28-77, T28-94 and T28-71.

Subclones of fragments of cDNA clone T28-77 were prepared in M13 phage and sequenced by the dideoxynucleotide termination procedure described by Sanger.

In all, a region of 3000 contiguous bases have been sequenced. This region contains the sequences which encode the two structural proteins VP-1 and VP-3 of HAV; and the other proteins VP-2, and VP-4. The proteins VP-1 and VP-3 are the major antigenic proteins; VP-2 and VP-4 are thought to be less valuable as antigens.

The amino acid sequence of a mixture of cyanogen bromide cleavage fragments generated from purified VP-3 protein (covered in copending USSN 541,836, supra) has been compared to the translation of

the DNA sequence to more precisely locate the region which encodes the VP-3.

The amino acid sequence of a mixture of cyanogen bromide cleavage fragments generated from purified VP-1 protein (covered in copending USSN 541,836, supra) has been compared to the translation of the DNA sequence to more precisely locate the region which encodes the VP-1 protein.

Referring to Fig. 1, the restriction map for the HAV genome indicates the location of the following 4 restriction sites: the Hinc II cleavage site at base 1643 of the DNA sequence; the Hind III cleavage site at base 1744; the Bam HI cleavage site at base 2049; and the Pvu II cleavage site at base 2722. These genome map positions are in the 5' to 3' direction on the genome: The nucleotide sequence of the region encoding the structural proteins, together with the reading frame for the protein is as follows:

```
                                27                                  54
GAT GTG TGG GAC GTC ACC TTG CAG TGT AAA CTT GGC TCT CAT GAA CCT CTT TGA
Asp Val Trp Asp Val Thr Leu Gln Cys Lys Leu Gly Ser His Glu Pro Leu  .

                                81                                 108
TCT TCC ACA AGG GGT AGG CTA CGG GTG AAA CCT CTT AGG CTA ATA CTT CTA TGA
Ser Ser Thr Arg Gly Arg Leu Arg Val Lys Pro Leu Arg Leu Ile Leu Leu  .

                               135                                 162
AGA GAT GCT TTG GAT AGG GCA ACA GCG GCG GAT ATT GGT GAG TTG TTA AGA CAA
Arg Asp Ala Leu Asp Arg Ala Thr Ala Ala Asp Ile Gly Glu Leu Leu Arg Gln

                               189                                 216
AAA CCA TTC AAC GCC GGA GGA CTG GCT CTC ATC CAG TGG ATG CAT TGA GTG GAT
Lys Pro Phe Asn Ala Gly Gly Leu Ala Leu Ile Gln Trp MET His  .  Val Asp

                               243                                 270
TGA TTG TCA GGG CTG TCT CTA GGT TTA ATC TCA GAC CTC TCT GTG CTT AGG GCA
 .  Leu Ser Gly Leu Ser Leu Gly Leu Ile Ser Asp Leu Ser Val Leu Arg Ala

                               297                                 324
AAC ACC ATT TGG CCT TAA ATG GGA TCC TGT GAG AGG GGG TCC CTC CAT TGA CAG
Asn Thr Ile Trp Pro  .  MET Gly Ser Cys Glu Arg Gly Ser Leu His  .  Gln

                               351                                 378
CTG GAC TGT TCT TTG GGG CCT TAT GTG GTG TTT GCC TCT GAG GTA CTC AGG GGC
Leu Asp Cys Ser Leu Gly Pro Tyr Val Val Phe Ala Ser Glu Val Leu Arg Gly

                               405                                 432
ATT TAG GTT TTT CCT CAT TCT TAA ACA ATA ATG AAT ATG TCC AAA CAA GGA ATT
Ile  .  Val Phe Pro His Ser  .  Thr Ile MET Asn MET Ser Lys Gln Gly Ile

                               459                                 486
TTC CAG ACT GTC GGG AGT GGC CTT GAC CAC ATC CTG TCT TTG GCA GAT ATT GAG
Phe Gln Thr Val Gly Ser Gly Leu Asp His Ile Leu Ser Leu Ala Asp Ile Glu
```

513 540
GAA GAG CAA ATG ATT CAG TCC GTT GTT AGG ACT GCA GTG ACT GGT GCT TCT TAT
Glu Glu Gln MET Ile Gln Ser Val Val Arg Thr Ala Val Thr Gly Ala Ser Tyr

567 594
TTT ACT TCT GTG GAC CAA TCT TCA GTT CAT ACT GCT GAG GTT GGC TTA CAT CAA
Phe Thr Ser Val Asp Gln Ser Ser Val His Thr Ala Glu Val Gly Leu His Gln

621 648
ATT GAA CCC TTG AAA ACC TCT GTT GAT AAA CCT AGT TCT AAG AAG ACT CAG GGG
Ile Glu Pro Leu Lys Thr Ser Val Asp Lys Pro Ser Ser Lys Lys Thr Gln Gly

675 702
GAG AAG TTT TTC CTG ATT CAT TCT GCT GAT TGG CTC ACT ACA CAT GCT CTA TTT
Glu Lys Phe Phe Leu Ile His Ser Ala Asp Trp Leu Thr Thr His Ala Leu Phe

729 756
CAT GAA GTT GCA AAA TTG GAC GTG GTG AAA TTA TTG TAT AAT GAG CAG TTT GCC
His Glu Val Ala Lys Leu Asp Val Val Lys Leu Leu Tyr Asn Glu Gln Phe Ala

783 810
GTC CAA GGT TTG TTG AGA TAC CAC ACA TAT GCA AGA TTT GGC ATT GAG ATT CAA
Val Gln Gly Leu Leu Arg Tyr His Thr Tyr Ala Arg Phe Gly Ile Glu Ile Gln

837 864
GTT CAG ATA AAT CCC ACA CCC TTT CAG CAA GGG GGG CTA ATT TGT GCT ATG GTT
Val Gln Ile Asn Pro Thr Pro Phe Gln Gln Gly Gly Leu Ile Cys Ala MET Val

891 918
CCT AGT GAC CAA AGT TAT GGT TCG ATA GCA TCC TTG ACT GTT TAT CCT CAT GGT
Pro Ser Asp Gln Ser Tyr Gly Ser Ile Ala Ser Leu Thr Val Tyr Pro His Gly

945 972
TTG TTA AAT TGC AAC ATT AAC AAT GTG GTT AGA ATA AAG GTT CCA TTT ATT TAT
Leu Leu Asn Cys Asn Ile Asn Asn Val Val Arg Ile Lys Val Pro Phe Ile Tyr

```
                                        999                                1026
ACT AGA GGT GCT TAT CAC TTT AAG GAT CCA CAG TAT CCA GTT TGG GAA TTA ACA
Thr Arg Gly Ala Tyr His Phe Lys Asp Pro Gln Tyr Pro Val Trp Glu Leu Thr

                                       1053                                1080
ATC AGA GTT TGG TCA GAG TTG AAT ATT GGA ACA GGA ACT TCA GCT TAC ACT TCA
Ile Arg Val Trp Ser Glu Leu Asn Ile Gly Thr Gly Thr Ser Ala Tyr Thr Ser

                                       1107                                1134
CTT AAT GTT TTA GCT AGG TTT ACA GAT TTG GAG TTA CAT GGA TTA ACT CCT CTT
Leu Asn Val Leu Ala Arg Phe Thr Asp Leu Glu Leu His Gly Leu Thr Pro Leu

                                       1161                                1188
TCT ACA CAG ATG ATG AGA AAT GAA TTT AGA GTT AGT ACT ACT GAA AAT GTT GTA
Ser Thr Gln MET MET Arg Asn Glu Phe Arg Val Ser Thr Thr Glu Asn Val Val

                                       1215                                1242
AAT TTG TCG AAT TAT GAA GAT GCA AGG GCA AAA ATG TCT TTT GCT TTG GAT CAG
Asn Leu Ser Asn Tyr Glu Asp Ala Arg Ala Lys MET Ser Phe Ala Leu Asp Gln

                                       1269                                1296
GAA GAT TGG AAG TCT GAT CCT TCC CAA GGT GGT GGA ATT AAA ATT ACT CAT TTT
Glu Asp Trp Lys Ser Asp Pro Ser Gln Gly Gly Gly Ile Lys Ile Thr His Phe

                                       1323                                1350
ACT ACC TGG ACA TCC ATT CCA ACC TTA GCT GCT CAG TTT CCA TTC AAT GCT TCA
Thr Thr Trp Thr Ser Ile Pro Thr Leu Ala Ala Gln Phe Pro Phe Asn Ala Ser

                                       1377                                1404
GAT TCG GTT GGA CAA CAA ATT AAA GTT ATT CCA GTG GAC CCA TAT TTT TTC CAG
Asp Ser Val Gly Gln Gln Ile Lys Val Ile Pro Val Asp Pro Tyr Phe Phe Gln

                                       1431                                1458
ATG ACA AAC ACC AAT CCT GAT CAA AAG TGT ATA ACT GCC TTG GCT TCT ATT TGT
MET Thr Asn Thr Asn Pro Asp Gln Lys Cys Ile Thr Ala Leu Ala Ser Ile Cys
```

1485 1512
CAG ATG TTT TGC TTT TGG AGG GGA GAT CTT GTT TTT GAT TTT CAG GTT TTT CCA
Gln MET Phe Cys Phe Trp Arg Gly Asp Leu Val Phe Asp Phe Gln Val Phe Pro

1539 1566
ACC AAA TAT CAT TCA GGT AGG TTG TTG TTT TGC TTT GTT CCT GGG AAT GAG TTG
Thr Lys Tyr His Ser Gly Arg Leu Leu Phe Cys Phe Val Pro Gly Asn Glu Leu

1593 1620
ATA GAT GTT ACT GGA ATC ACA TTA AAA CAG GCA ACC ACT GCT CCT TGT GCA GTG
Ile Asp Val Thr Gly Ile Thr Leu Lys Gln Ala Thr Thr Ala Pro Cys Ala Val

1647 1674
ATG GAC ATT ACA GGA GTG CAG TCA ACC TTG AGA TTT CGT GTT CCT TGG ATT TCT
MET Asp Ile Thr Gly Val Gln Ser Thr Leu Arg Phe Arg Val Pro Trp Ile Ser

1701 1728
GAT ACA CCC TAT CGA GTG AAT AGG TAC ACG AAG TCA GCA CAT CAA AAA GGT GAG
Asp Thr Pro Tyr Arg Val Asn Arg Tyr Thr Lys Ser Ala His Gln Lys Gly Glu

1755 1782
TAT ACT GCC ATT GGG AAG CTT ATT GTG TAT TGT TAT AAT AGG CTG ACT TCT CCT
Tyr Thr Ala Ile Gly Lys Leu Ile Val Tyr Cys Tyr Asn Arg Leu Thr Ser Pro

1809 1836
TCT AAT GTT GCT TCT CAT GTT AGA GTT AAT GTT TAT CTT TCA GCA ATT AAT TTG
Ser Asn Val Ala Ser His Val Arg Val Asn Val Tyr Leu Ser Ala Ile Asn Leu

1863 1890
GAA TGT TTT GCT CCT CTT TAT CAT GCT ATG GAT GTT ACC ACA CAG GTT GGA GAT
Glu Cys Phe Ala Pro Leu Tyr His Ala MET Asp Val Thr Thr Gln Val Gly Asp

1917 1944
GAT TCA GGA GGT TTT TCA ACA ACA GTT TCG ACA GAG CAG AAT GTT CCT GAT CCC
Asp Ser Gly Gly Phe Ser Thr Thr Val Ser Thr Glu Gln Asn Val Pro Asp Pro

1971 1998
CAA GTT GGT ATA ACA ACT ATG AAG GAC CTG AAA GGG AAA GCC AAT AGG GGA AAG
Gln Val Gly Ile Thr Thr MET Lys Asp Leu Lys Gly Lys Ala Asn Arg Gly Lys

2025 2052
ATG GAT GTT TCA GGA GTG CAA GCA CCT GTG GGA GCT ATC ACA ACA ATT GAG GAT
MET Asp Val Ser Gly Val Gln Ala Pro Val Gly Ala Ile Thr Thr Ile Glu Asp

2079 2106
CCA GCA TTA GCA AAG AAA GTA CCT GAA ACG TTT CCT GAA TTG AAG CCT GGA GAG
Pro Ala Leu Ala Lys Lys Val Pro Glu Thr Phe Pro Glu Leu Lys Pro Gly Glu

2133 2160
TCT AGA CAT ACA TCA GAT CAC ATG TCT ATT TAT AAA TTC ATG GGA AGG TCT CAT
Ser Arg His Thr Ser Asp His MET Ser Ile Tyr Lys Phe MET Gly Arg Ser His

2187 2214
TTT TTG TGT ACT TTT ACC TTC AAT TCA AAT AAT AAA GAG TAC ACA TTT CCA ATA
Phe Leu Cys Thr Phe Thr Phe Asn Ser Asn Asn Lys Glu Tyr Thr Phe Pro Ile

2241 2268
ACC TTG TCT TCG ACT TCT AAT CCT CCT CAT GGT TTA CCA TCA ACA TTA AGG TGG
Thr Leu Ser Ser Thr Ser Asn Pro Pro His Gly Leu Pro Ser Thr Leu Arg Trp

2295 2322
TTC TTC AAT CTG TTT CAG TTG TAT AGA GGA CCA TTG GAT TTG ACA ATT ATC ATC
Phe Phe Asn Leu Phe Gln Leu Tyr Arg Gly Pro Leu Asp Leu Thr Ile Ile Ile

2349 2376
ACA GGA GCT ACT GAT GTG GAT GGA ATG GCC TGG TTT ACT CCA GTA GGC CTT GCT
Thr Gly Ala Thr Asp Val Asp Gly MET Ala Trp Phe Thr Pro Val Gly Leu Ala

2403 2430
GTT GAC ACC CCA TGG GTG GAA AAG GAA TCA GCT TTG TCT ATT GAT TAT AAA ACT
Val Asp Thr Pro Trp Val Glu Lys Glu Ser Ala Leu Ser Ile Asp Tyr Lys Thr

```
                                        2457                                   2484
GCC CTT GGA GCT GTT AGA TTT AAT ACA AGA AGA ACA GGG AAC ATT CAG ATT AGA
Ala Leu Gly Ala Val Arg Phe Asn Thr Arg Arg Thr Gly Asn Ile Gln Ile Arg

                                        2511                                   2538
TTG CCA TGG TAT TCT TAT TTA TAT GCT GTG TCT GGA GCA CTG GAT GGC TTG GGA
Leu Pro Trp Tyr Ser Tyr Leu Tyr Ala Val Ser Gly Ala Leu Asp Gly Leu Gly

                                        2565                                   2592
GAT AAG ACA GAT TCT ACA TTT GGA TTG GTT TCC ATA CAG ATT GCA AAT TAC AAC
Asp Lys Thr Asp Ser Thr Phe Gly Leu Val Ser Ile Gln Ile Ala Asn Tyr Asn

                                        2619                                   2646
CAC TCT GAT GAA TAT TTG TCC TTT AGT TGT TAT TTG TCT GTC ACA CAA CAA TCA
His Ser Asp Glu Tyr Leu Ser Phe Ser Cys Tyr Leu Ser Val Thr Gln Gln Ser

                                        2673                                   2700
GAG TTC TAT TTT CCT AGA GCT CCA TTA AAT TCA AAT GCT ATG TTG TCC ACT GAG
Glu Phe Tyr Phe Pro Arg Ala Pro Leu Asn Ser Asn Ala MET Leu Ser Thr Glu

                                        2727                                   2754
TCT ATG ATG AGT AGA ATT GCA GCT GGA GAC TTG GAG TCA TCA GTG GAT GAT CCT
Ser MET MET Ser Arg Ile Ala Ala Gly Asp Leu Glu Ser Ser Val Asp Asp Pro

                                        2781                                   2808
AGA TCA GAG GAA GAC AGA AGA TTT GAG AGT CAT ATA GAA TGT AGG AAA CCA TAT
Arg Ser Glu Glu Asp Arg Arg Phe Glu Ser His Ile Glu Cys Arg Lys Pro Tyr

                                        2835                                   2862
AAA GAA TTG AGA TTG GAG GTT GGG AAA CAA AGA CTT AAA TAT GCT CAG GAA GAG
Lys Glu Leu Arg Leu Glu Val Gly Lys Gln Arg Leu Lys Tyr Ala Gln Glu Glu

                                        2889                                   2916
TTG TCA AAT GAA GTG CTT CCA CCT CCT AGG AAA ATG AAG GGG TTA TTT TCA CAA
Leu Ser Asn Glu Val Leu Pro Pro Pro Arg Lys MET Lys Gly Leu Phe Ser Gln

                                        2943                                   2970
GCC AAA ATT TCT CTT TTT TAT ACT GAG GAA CAT GAA ATA ATG AAA TTT LCQ TGG
Ala Lys Ile Ser Leu Phe Tyr Thr Glu Glu His Glu Ile MET Lys Phe     Trp

                                        2997
AGA GGA GTG A
Arg Gly Val
```

Within this entire sequence is encoded the information necessary to make the antigenic proteins of HAV. The sequences encoding for the structural proteins begin at base 403. The VP-3 protein is encoded starting approximately at map position 1149. The VP-I protein is encoded starting approximately at map position 1882. The key sub-unit sequences within VP-1, designated Sequences I, II, III, IV, and V, start, respectively at 1882, 1963, 1999, 2146, 2347. An effective vaccine can contain any one or more of these subunits, or of any other effective peptide subunit encoded within the genome sequence.

The value of the nucleotide sequence will be readily apparent to those skilled in the art: a particular nucleotide sequence encoding for an active peptide or peptide fragment can be synthesized and cloned into an expression system in order to produce the particular peptide or subunit desired. These nucleotide sequences can be either made by total synthesis, that is, by linking bases according to known techniques, or by direct isolation from the viral RNA or cDNA. If the start of the nucleotide sequence desired does not correspond to the particular peptide, known techniques which enzymatically add or subtract the particular bases to the nucleotide chain can be employed to precisely make the nucleotide sequence.

Other nucleotide sequences which are valuable as encoding antigenic proteins are the sequences from base 1749 to base 2722; from base 1487 to base 2980 and from base 1644 to base 2722, all contained within the above sequence. The sequence from base 1749 to base 2722 is especially valuable as a vector for producing antigen protein, see the following examples.

EXAMPLE 7
EXPRESSION OF cDNA CLONES

In order to begin studies on expression, a DNA clone containing the region of sequences encoding VP-3 and VP-1 in a single insert was first constructed. The DNA of clone 28-94 was cleaved with EcoRI and Xba I restriction enzymes and the 5.76 kb band of DNA containing the 5' portion of the viral structural protein genes was purified by agarose gel electrophoresis and electroelution. Similarly, the 2.3 kb EcoRI-XbaI band containing the 3'portion of the viral structural genes of clone 28-77 was purified. These two fragments were ligated together to re-form pBR322 vector molecules containing the entire non-rearranged viral structural gene sequences. The ligated mixture was transformed into E. coli strain RR-1, thereby generating clone 57-5 with a plasmid containing an insert of intact VP-3 to VP-1 information.

The expression of HAV-specified proteins containing antigenic reactivity to anti-HAV antibodies can be accomplished in bacterial cells, yeast, or higher eucaryotic cells. Some of the possible methods to obtain expression of the cDNA clones into proteins or polypeptide products are described below.

In the first example, fragments of HAV cDNA are inserted downstream from the bacterial lac promoter making a fusion product with the N-terminal amino acids of the bacterial β-galactosidase followed by HAV polypeptides. The fragments of HAV cDNA used having ends generated by Bgl II or Bam H1 or Hind III or Pst I or Taq I or Sau 3A or Nco I are ligated into the appropriate sites of the known expression plasmids pUC 7, 8, or 9 or pCQV2. The expression plasmid used with each HAV DNA fragment is selected to give an open reading frame for translation after insertion of the DNA. The recombinant expression plasmids are transformed into E. coli and appropriate clones are selected, grown to an optical density of 0.8 at a wavelength of 550, and lysed. The cellular extract is then used for detection of HAV-specific antigenic peptides.

Alternatively, the fragments of HAV cDNA are inserted downstream of the Herpes simplex viral thymidine kinase gene promoter in a plasmid, cloned in E. coli and the resulting cloned recombinant plasmid is transfected into mouse L cells, mouse 3T3 cells, or other eukaryotic cells in culture. The fragments of HAV cDNA used having ends generated by Bgl II or Bam H1 or Hind III or Bal 31 enzymes are ligated behind the thymidine kinase ATG translational start codon at the Rsa I site using appropriate linker molecules of DNA to make the connection in frame with respect to translation. The eucaryotic cells transfected by the recombinant expression plasmids are selected by cotransfection with the entire Herpes thymidine kinase gen and subsequent growth in HAT selection medium. The colonies of selected cells are grown and cellular extracts prepared for detection of HAV-specific antigenic

EXAMPLE 8
DETECTION OF EXPRESSED HAV PROTEIN AND POLYPEPTIDES

The cells expressing HAV proteins or polypeptides are labeled by growth in the presence of $^{35}$S-labeled methionine or $^{14}$C-labeled leucine. Extracts of the cells are then prepared and reacted with anti-HAV antiserum or anti-HAV monoclonal antibodies. The antigen-antibody complexes are collected by precipitation with formalin fixed Staph A, dissociated by boiling in SDS-EDTA, and separated by SDS-polyacrylamide gel electrophoresis. The proteins are then visualized by autoradiography.

Alternatively, the expressed HAV proteins or polypeptides are detected by a competition radioimmune assay. Purified HAV virion particles are labeled in vitro with $^{125}$I and precipitated with anti-HAV antiserum or anti-HAV monoclonal antibodies. This precipitation is performed in the presence of increasing amounts of unlabeled extracts prepared from the expression cells. The antigen-antibody complexes are collected on filters and counted in a gamma-counter. The presence of expressed HAV products in the expression cells is shown by a decrease in counts as the amount of cellular extract is increased in the assay.

EXAMPLE 9
EXPRESSION OF HAV SEQUENCES IN E. COLI
Construction of Expression Plasmids

(1) Approximately 0.5 μg of pCQV2 (Cary Queen) DNA was cleaved with BamHI and PvuII and treated

with phosphatase and ligated to 2 μg of gel purified 675 bp long fragment derived from clone T28-123 by cleavage with BamHI and PvuII. The ligated mixture was transformed into HB101 cells and ampicillin resistant clones were screened for the presence of insert with purified nick translated 675 base pair fragment. One positive clone designated pHAV-6 was assayed for production of HAV specified proteins. The junction sequence between pCQV2 and the insert was confirmed by cleavage with BamHI and PvuII which released the appropriate fragment.

(2) Clone 57-5 (prepared by coupling cDNA clone 28-94 with cDNA clone 28-77) was used get expression of almost the entire VP-1 gene and the C-terminal region of VP-3. DNA from plasmid 57-5 was cleaved with BglII and PvuII and 1.12 kbp fragment was purified by gel electrophoresis. Approximately 2 μg of the fragment was ligated to 0.5 μg of BamHI-PvuII cleaved pCQV2 DNA and the ligated mixture used to transform HB101 cells. Ampicillin resistant clones were screened by hybridization to nick translated BamHI-PvuII cleaved fragment. One of the clones which proved positive was designated pHAV-57-11 and was assayed for expression of VP3 and/or VP1.

Both of these constructions allow read through from the initiating AUG codon within pCQV2 and continues beyond the end of the insert into the pCQV2 sequences for 50 amino acids before a termination codon is encountered. The predicted open reading frame of pHAV-6 is therefore 275 amino acid residues and that of pHAV57-11 is 464 amino acid residues. Plasmid HAV57-11 contains the following nucleic acid sequences:

1. GAT CTT GTT TTG ATT TTT CAG GTT TTT.

2. GTT GGA GAT GAT TCA GGA GGT TTT TCA ACA ACA.

3. ATG AAG GAC CTG AAA GGG AAA GCC AAT AGG GGA AAG.

4. ATG GAT GTT TCA GGA GTG CAA GCA CCT GTG GGA GCT
ATC ACA ACA ATT GAG GAT CCA GCA TTA GCA AAG AAA GTA
CCT GAA ACG TTT.

5. ATG GGA AGG TCT CAT TTT TTG TGT ACT TTT ACC TTC
AAT TCA AAT AAT AAA GAG TAC.

6. ATG GCC TGG TTT ACT CCA GTA GGC CTT GCT GTT GAC
ACC CCA.

Plasmid HAV-6 contains the nucleic acid sequences identified by numbers 5 and 6 above, but not those sequences identified by numbers 1, 2, 3 and 4.

Detection of Expressed Proteins

Expression plasmids pHAV-6 and pHAV57-11 were grown in L-broth to log phase and induced by heat shock at 42° C. The pCQV2 expression vector carries a temperature sensitive λ repressor which suppresses synthesis of proteins inserted at the λ rightward promoter ($\lambda P_R$). Heating at 42° C inactivated the repressor and allowed the expression of genes located downstream from $\lambda P_R$. 1 ml of cells were pelleted before induction and 30 minutes after induction, lysed by boiling with SDS mercaptoethanol and the proteins separated by discontinuous gel electrophoresis. The proteins were transferred either to nitrocellulose (BA-85) paper or to Millipore filters (HAHY grade) by electroblotting. The proteins that were transferred were probed with two polyclonal antihepatitis A antisera (antiHAV) designated DB-2 and C-149 raised by injection of SDS-heat denatured virus into rabbits.

The antisera were in turn detected by $I^{125}$ labelled protein A. Two criteria were used to determine

expression of HAV specified proteins: (1) Heat inducibility, and (2) Appearance of a novel band absent in the original vector pCQV2 when probed with post-immune serum. Plasmid HAV-6 showed a unique band at ~ 30 and 20 k daltons when probed with DB-2 serum and a band at 30 kd when probed with C149 post immune serum. Since pHAV-6 contains an insert entirely within VP-1 coding region, expression of VP-1 segments in E. coli was achieved.

Plasmid HAV57-11 showed six prominent bands when probed with DB-2 serum. They are, in order of decreasing size, ~ 50, 35, 23, 20, 16, and 14 k daltons. Presumably, the smaller peptides represent degradation products of the ~ 50 kd protein. The profile is simpler when the probe used was C149. The 50 kd band was clearly visible as well as two weak bands around 30 k daltons. The small molecular weight protein bands were presumably degradation products. Since pHAV57-11 contains a DNA insert which encodes 133 amino acids of VP-3 representing the C-terminus as well as most of VP-1 sequences excluding ~ 30 amino acids from the C-terminal end expression of VP-1 segments and VP-3 segments in E. coli was achieved.

Screening with Marmoset Serum

Pre- and postimmune serum from a marmoset injected with native virus were used to screen the protein blots. Preimmune serum failed to bind to any novel induced protein in both pHAV-6 and pHAV57-11. Postimmune serum on the other hand reacted with a protein ~ 50 kd in size produced in induced pHAV57-11 cells and another ~ 18k daltons in size. The detection of ~ 50 kd protein in pHAV57-11 suggests that at least some of the same determinants or epitopes seen on the native virus are also available for interaction with antibody in the 50 kd protein. The presence of ~ 133 amino acid residues of VP-3 sequences at the N-terminus of VP-1 presumably allows the VP-1 protein to assume a tertiary structure which exposes at least some of the determinants normally exposed on native virus. Hence the 50 kd protein and possibly the ~ 18 kd protein made in E. coli are potential immunogenes which may be capable of eliciting normal immune response.

## Claims

1. Vectors containing all or part of the following nucleotide sequences and adapted to express in a suitable host the peptide coded for by said nucleotide sequence :

GAT CTT GTT TTT GAT TTT CAG GTT TTT,
GTT GGA GAT GAT TCA GGA GGT TTT TCA ACA ACA,
ATG AAG GAC CTG AAA GGG AAA GCC AAT AGG GGA AAG,
ATG GAT GTT TCA GGA GTG CAA GCA CCT GTG GGA GCT ATC ACA ACA ATT GAG GAT CCA GCA TTA GCA AAG AAA GTA CCT GAA ACG TTT,
ATG GGA AGG TCT CAT TTT TTG TGT ACT TTT ACC TTC AAT TCA AAT AAT AAA GAG TAC and
ATG GCC TGG TTT ACT CCA GTA GGC CTT GCT GTT GAC ACC CCA.

2. The vectors of claim 1, wherein the host is a prokaryotic or eukaryotic organism.

3. A suitable prokaryotic or eukaryotic host containing a vector according to claim 2, the host adapted to express at least part of the peptide coded for by said nucleotide sequence.

4. Plasmid HAV 57-11 containing the nucleic acid sequences

GAT CTT GTT TTT GAT TTT CAG GTT TTT,
GTT GGA GAT GAT TCA GGA GGT TTT TCA ACA ACA,
ATG AAG GAC CTG AAA GGG AAA GCC AAT AGG GGA AAG,
ATG GAT GTT TCA GGA GTG CAA GCA CCT GTG GGA GCT ATC
ACA ACA ATT GAG GAT CCA GCA TTA GCA AAG AAA GTA CCT
GAA ACG TTT,
ATG GGA AGG TCT CAT TTT TTG TGT ACT TTT ACC TTC AAT
TCA AAT AAT AAA GAG TAC and
ATG GCC TGG TTT ACT CCA GTA GGC CTT GCT GTT GAC ACC
CCA.

5. Plasmid HAV-6 containing the nucleic acid sequences

ATG GGA AGG TCT CAT TTT TTG TGT ACT TTT ACC TTC AAT
TCA AAT AAT AAA GAG TAC and
ATG GCC TGG TTT ACT CCA GTA GGC CTT GCT GTT GAC ACC
CCA.

## Revendications

1. Vecteurs contenant la totalité ou une partie des séquences nucléotidiques suivantes et adaptés pour exprimer chez un hôte approprié le peptide pour lequel code ladite séquence nucléotidique

GAT CTT GTT TTT GAT TTT CAG GTT TTT,
GTT GGA GAT GAT TCA GGA GGT TTT TCA ACA ACA,
ATG AAG GAC CTG AAA GGG AAA GCC AAT AGG GGA AAG,
ATG GAT GTT TCA GGA GTG CAA GCA CCT GTG GGA GCT ATC
ACA ACA ATT GAG GAT CCA GCA TTA GCA AAG AAA GTA CCT
GAA ACG TTT,
ATG GGA AGG TCT CAT TTT TTG TGT ACT TTT ACC TTC AAT
TCA AAT AAT AAA GAG TAC et
ATG GCC TGG TTT ACT CCA GTA GGC CTT GCT GTT GAC ACC
CCA.

2. Vecteurs de la revendication 1, dans lesquels l'hôte est un organisme prokaryotique ou eukaryotique.

3. Hôte prokaryotique ou eukaryotique approprié contenant un vecteur selon la revendication 2, hôte adapté pour exprimer au moins une partie du peptide pour lequel code ladite séquence nucléotidique.

4. Plasmide HAV57-11 contenant les séquences d'acide nucléique

GAT CTT GTT TTT GAT TTT CAG GTT TTT,
GTT GGA GAT GAT TCA GGA GGT TTT TCA ACA ACA,
ATG AAG GAC CTG AAA GGG AAA GCC AAT AGG GGA AAG,
ATG GAT GTT TCA GGA GTG CAA GCA CCT GTG GGA GCT ATC
ACA ACA ATT GAG GAT CCA GCA TTA GCA AAG AAA GTA CCT
GAA ACG TTT,
ATG GGA AGG TCT CAT TTT TTG TGT ACT TTT ACC TTC AAT
TCA AAT AAT AAA GAG TAC et
ATG GCC TGG TTT ACT CCA GTA GGC CTT GCT GTT GAC ACC
CCA.

5. Plasmide HAV-6 contenant les séquences d'acide nucléique

ATG GGA AGG TCT CAT TTT TTG TGT ACT TTT ACC TTC AAT
TCA AAT AAT AAA GAG TAC et
ATG GCC TGG TTT ACT CCA GTA GGC CTT GCT GTT GAC ACC
CCA.

## Ansprüche

1. Vektoren, die die folgenden Nucleotid-Sequenzen ganz oder teilweise enthalten und in einem geeigneten Wirt das durch diese Nucleotid-Sequenz kodierte Peptid exprimieren können:

GAT CTT GTT TTT GAT TTT CAG GTT TTT,
GTT GGA GAT GAT TCA GGA GGT TTT TCA ACA ACA,
ATG AAG GAC CTG AAA GGG AAA GCC AAT AGG GGA AAG,
ATG GAT GTT TCA GGA GTG CAA GCA CCT GTG GGA GCT ATC
ACA ACA ATT GAG GAT CCA GCA TTA GCA AAG AAA GTA CCT
GAA ACG TTT,
ATG GGA AGG TCT CAT TTT TTG TGT ACT TTT ACC TTC AAT
TCA AAT AAT AAA GAG TAC und
ATG GCC TGG TTT ACT CCA GTA GGC CTT GCT GTT GAC ACC
CCA.

2. Vektoren nach Anspruch 1, worin der Wirt ein prokaryotischer oder eukaryotischer Organismus ist.

3. Geeigneter prokaryotischer oder eukaryotischer Wirt, der einen Vektor nach Anspruch 2 enthält, wobei der Wirt wenigstens einen Teil des durch diese Nucleotid-Sequenz kodierten Peptids exprimieren kann.

**4.** Plasmid HAS 57-11, das die Nucleinsäure-Sequenzen enthält:

GAT CTT GTT TTT GAT TTT CAG GTT TTT,
GTT GGA GAT GAT TCA GGA GGT TTT TCA ACA ACA,
ATG AAG GAC CTG AAA GGG AAA GCC AAT AGG GGA AAG.
ATG GAT GTT TCA GGA GTG CAA GCA CCT GTG GGA GCT ATC ACA ACA ATT GAG GAT CCA GCA TTA GCA AAG AAA GTA CCT GAA ACG TTT,
ATG GGA AGG TCT CAT TTT TTG TGT ACT TTT ACC TTC AAT TCA AAT AAT AAA GAG TAC und
ATG GCC TGG TTT ACT CCA GTA GGC CTT GCT GTT GAC ACC CCA.

**5.** Plasmid HAV-6, das die Nucleinsäure-Sequenzen enthält:

ATG GGA AGG TCT CAT TTT TTG TGT ACT TTT ACC TTC AAT TCA AAT AAT AAA GAG TAC und
ATG GCC TGG TTT ACT CCA GTA GGC CTT GCT GTT GAC ACC CCA.

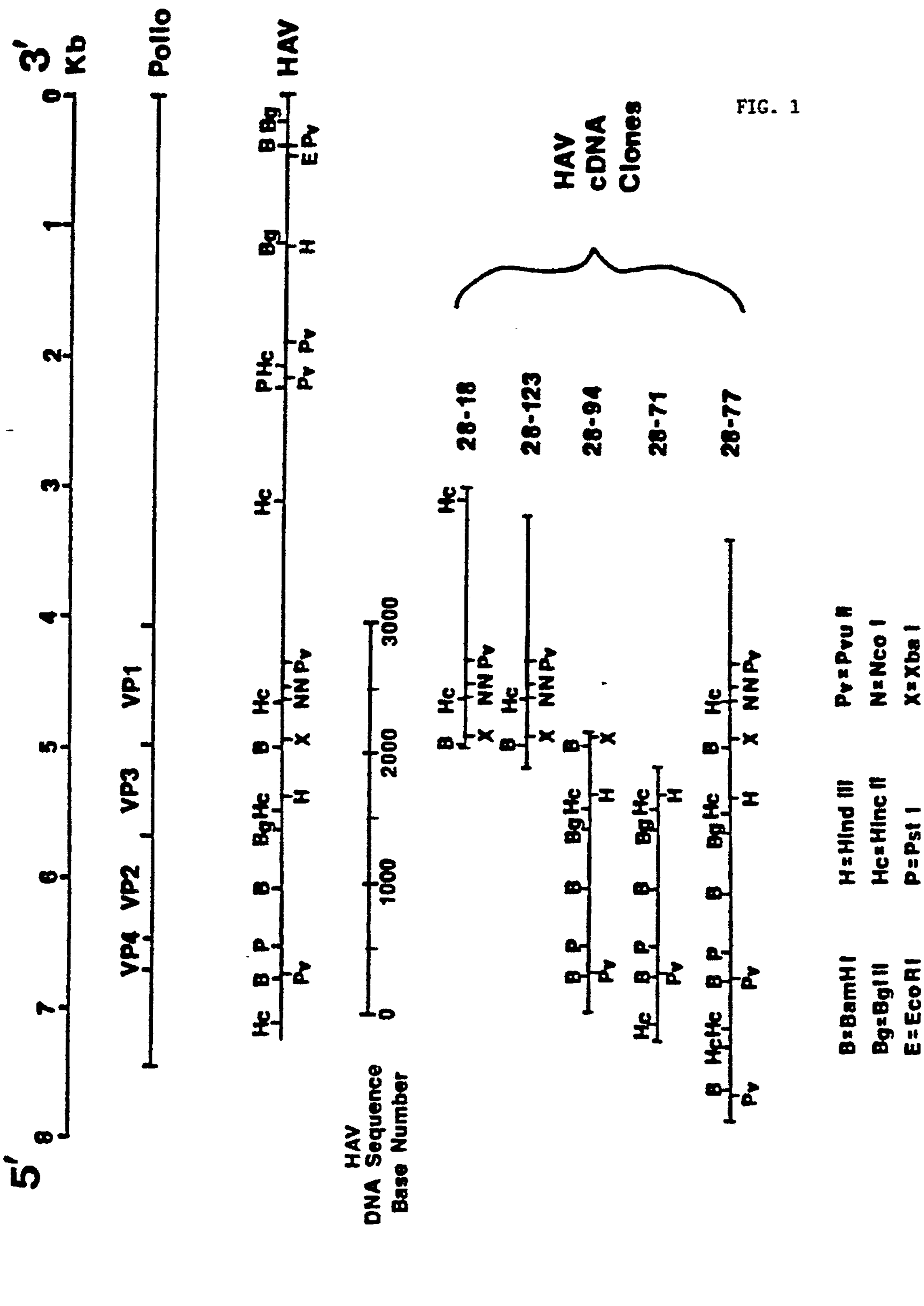
5'
3'
Kb
8
7
6
5
4
3
2
1
0
Polio
VP4
VP2
VP3
VP1
HAV
HAV DNA Sequence Base Number
0
1000
2000
3000
HAV cDNA Clones
28-18
28-123
28-94
28-71
28-77
B=BamHI
Bg=BglII
E=EcoRI
H=HindIII
Hc=HincII
P=PstI
Pv=PvuII
N=NcoI
X=XbaI

FIG. 1